# EUROPEAN PATENT APPLICATION

(11) **EP 4 094 756 A1**
(43) Date of publication of application: **30.11.2022**
(21) Application number: 21205924.0
(22) Date of filing: 02.11.2021
(51) Int. Cl.: A61K 31/122, A61K 9/00, A61K 36/38, A61P 31/14

(54) **COMPOSITIONS FOR TREATING SARS-COV2 INFECTION**

(30) Priority: 26.05.2021 EP 21175949
(71) Applicant: Hirsch-Apotheke, Wolfgang Scholz e.K., 58507 Lüdenscheid (DE)
(72) Inventor: SCHOLZ, Wolfgang, 58507 Lüdenscheid (DE); LUDWIG, Stefan, 48161 Münster (DE); HEMPEL, Georg, 40474 Düsseldorf (DE); HENSEL, Andreas, 48161 Münster (DE)
(74) Representative: Schiweck Weinzierl Koch Patentanwälte Partnerschaft mbB

(57) **Abstract**

The present invention relates to compositions comprising hypericin and/or pseudohypericin for use in a method for treating or preventing an infection with SARS-CoV2 in a human subject in need thereof. The present invention further relates to (mouth wash, gargle solution, nose spray, inhalant, lozenge, tablet, chewing gum, injection solution comprising hypericin and/or pseudohypericin.

## Description

The present invention relates to compositions comprising hypericin and/or pseudohypericin for use in a method for treating or preventing an infection with SARS-CoV2 in a human subject in need thereof. The present invention further relates to (mouth wash, gargle solution, nose spray, inhalant, lozenge, tablet, chewing gum, injection solution comprising hypericin and/or pseudohypericin.

The novel coronavirus disease (COVID-19) is caused by the severe acute respiratory syndrome coronavirus 2 (SARS-CoV-2), a beta coronavirus that was earlier identified in late December 2019 in the city of Wuhan, China. Up to date, Coronavirus reached 153,343,066 cases with nearly 3,212,757 deaths (https://www.worldometers.info/coronavirus/), illustrating the morbidity and mortality rate in addition to the rapid transmission of the virus. Health and economic problems, lack of direct evidence on the virus origin, and absence of specific treatment constitute major challenges during facing the ongoing pandemic. SARS-CoV-2 is somehow similar to the SARS-CoV-1 and the Middle East respiratory syndrome coronavirus (MERS-CoV) in their genomic structure, where the structural proteins, as the spike (S), envelope (E), membrane (M), and nucleocapsid (N) are of major importance. The SARS CoV-2 infection starts when the viral S spike interacts with the ACE2 receptors on the surface of airway epithelial cells. The virus entry is then primed by the TMPRSS2 enzyme or other proteases, which cleave the S protein and initiate the virus fusion. The viral RNA ends up in the cell and viral replication is initiated.

There are several approaches that had been introduced to reveal potential antivirals against SARS CoV-2. Among them, (i) the usage of existing clinically approved drugs. Nevertheless, undesired side effects may occur in relation to different doses and pharmacological characteristics. The second practice is the computational drug screening while the third one is (iii) the development of new drugs that could inhibit the SARS CoV-2 life cycle, which, of course, is time consuming and expensive. It is worth mentioning that targeting the virus entry or virus survival in the environment result in prevention of transmission among individuals. Thus, such antivirals could be used as therapeutic and/ or prophylactic agents.

Another approach directed to viral infections incl. SARS-CoV2 (Bajrai et al., bioRxiv preprint Jan 31, 2021; doi: 10.1101/2021.01.11.426295) is applying natural plant materials in-vitro to screen for such plant materials which may be useful for fighting viral infections of cells. Among tested plants is Hypericum perforatum (H.P.), also known as Perforate St John's-wort. Extracts of H.P. were previously found to be effective against some viruses and bacteria, including human cytomegalovirus, human immunodeficiency syndrome virus (HIV), influenza A virus, and infectious bronchitis virus. Within the Hypericum perforatum (H.P.) extract, several ingredients could be found (e.g. hypericin and pseudohypericin, hyperforin, procyanidin C1, and quercetin 3-O glucuronide). H.P. was also used as topical treatment, immune modulator, and anti-cancer therapy. Procyanidin C1 was used earlier due to its antiviral and antioxidant activities. Meanwhile, quercetin 3-0 glucuronide could be used as an anti-inflammatory, antiviral, and antioxidative metabolite.

However, as has been shown previously (cf. Bajrai et al., loc. cit.), such preparations, particularly if they contain, apart from plant extracts also solid plant material, i.e., non-extracted plant material, or do not accord to state of the art pharmaceutical standards or official pharmacopoeas lead to toxicity already at low doses, thereby preventing an application and effective treatment in humans. The antiviral IC50-concentration reported in Bajrai et al., i.e., "the highest efficacy with IC50 of 1.56 mcg/ml" (see page 9, lines 199-200) is practically the same where per definition of the authors up to < 50% of the cells die due to toxicity observed (see page 8, lines 173-175: "more than 50% cell survival rate was considered to be the maximum non-toxic concentration" and; see also Table 3 on page 9 thereof). Based on such a toxicity and "no-therapeutic-window" scenario, it is highly doubtful whether increased concentrations representing important parameters such as IC95=ED95 or IC100=ED100 can be assessed in a reliable manner; evidently for that reason IC95 and IC100 values have not been reported, and therefore the therapeutic index (TI = LD5/ED95) cannot be determined which to know is the very essential precondition for any evaluation targeting at a clinical application in humans. In consistency with good therapeutic practices effective doses above doses which already cause toxicity cannot be administered. The study of Bajrai et al., loc. cit. thus teaches away from using H.P. preparations including plant extract and solid plant material for an effective treatment of SARS-CoV2, since the toxicity observed at low doses does not allow translation of H.P. preparations into effective doses.

This is so because, the dose at which an antiviral effect may have been observed and the dose at which toxicity on test cells occurred, coincide. Hence, there is in essence no therapeutic window, which is a precondition for applying a potential drug to treat humans.

In sum, from Bajrai et al. the skilled person learns that a preparation from Hypericum perforatum will not have a therapeutic value.

These and further disadvantages need to be overcome. The present invention therefore addresses these needs and technical objectives and provides a solution as described herein and as defined in the claims.

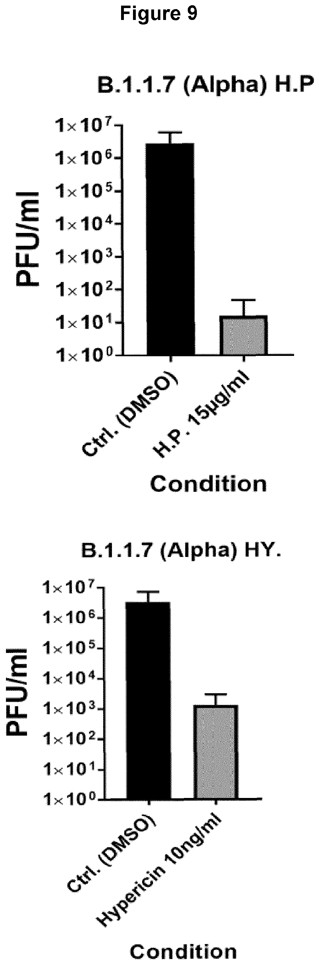

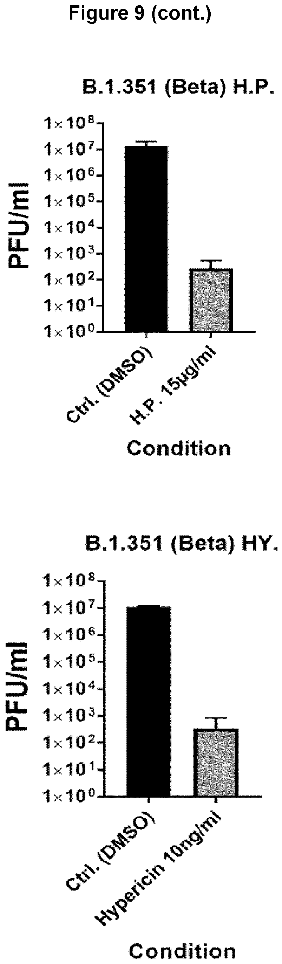

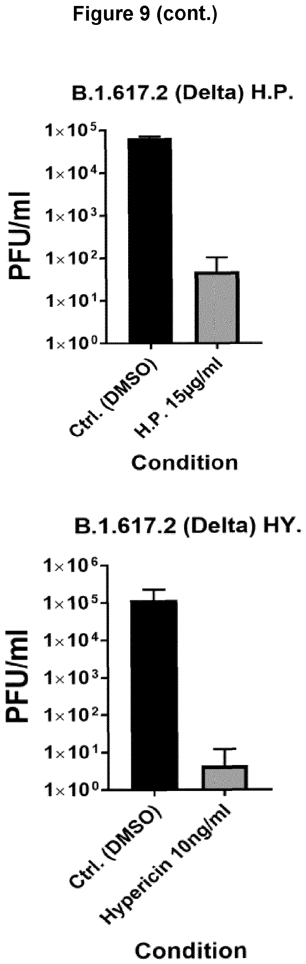

Accordingly, the present invention provides, inter alia, a broad range of dosings and concentrations which produce antiviral efficacy in a dose-dependent manner, shows a defined therapeutic window and allows to assess a usable therapeutic index (TI), since, e.g. toxic concentrations of the Hypericum perforatum (H.P.) extract or major ingredients such as hypericin are much higher than those needed for 100%-antiviral efficacy. That said, in contrast to the prior art, the present invention paves the way for using compositions comprising hypericin and/or pseudohypericin, e.g. as pure substances or in the form of an extract for treating or preventing an infection with SARS-CoV2 in a human subject in need thereof. It has also been investigated by the inventors that the compositions according to the present invention comprising hypericin and/or pseudohypericin, which possess antiviral activity against (wild type) SARS-CoV-2, also show surprisingly antiviral activity against emerged SARS-CoV-2 variants Alpha ("British variant"), Beta ("South African variant") and Delta ("Indian variant") (see Fig. 9). The European Centre for Disease Prevention and Control (EU/EEA) announced four categories for the variants-of-concern as of October 2021, which include (i) Variants of Concern (short: VOC), (ii) Variants of Interest (short: VOI), (iii) Variants under monitoring (short: VUM), and (iv) De-escalated variants (short: VDE). The members of VOC group had shown a significant impact on transmissibility, severity, or immunity and involve variants of Beta (B.1.351; first detected in South Africa), Gamma (P.1; first detected in Brazil), and Delta (B.1.617.2; first detected in India). On the other hand, De-escalated variants were recognized as ones that are no longer circulating or had minimum or no epidemiological impact, and include the British variant (Alpha; B.1.1.7).

It was further demonstrated that the compositions of the present invention also have an effect even without light, although it was assumed that light was needed (Hudson et al. (1993), Antiviral Research, 20: 173-178; Jacobson et al. (2001), Antimicrobial Agents and Chemotherapy, 45(2): 517-524).

Accordingly, the present invention relates to compositions comprising hypericin and/or pseudohypericin (preferably hypericin) for use in a method for treating or preventing an infection with SARS-CoV2 in a human subject in need thereof. Preferably, said composition does not comprise non-extracted H.P. (solid) plant material, such as aerial parts or substantial amounts of such aerial parts.

"SARS-CoV-2" when used herein refers to a wild-type SARS-CoV-2 strain. "Wild-type" in the context of SARS-CoV-2 refers to a strain of SARS-CoV-2 that contains no major mutations on genome level resulting in VOC, VOI, VUM, or VDE in comparison to the reference genome of the coronavirus that was identified in late December 2019 in the city of Wuhan, China. A preferred reference genome of a coronavirus in comparison to which mutations on genome level can be determined is SARS-CoV-2 Wuhan-Hu-1 strain (Accession NC_045512, Version NC_045512.2).

Preferably, the term "SARS-CoV-2" when used herein further, i.e., in addition to wild-type SARS-CoV-2, includes SARS-CoV-2 variants, such as Alpha, Beta and/or Delta SARS-CoV-2 variants; more preferably, the term "SARS-CoV-2" further, i.e., in addition to wild-type SARS-CoV-2, includes the SARS-CoV-2 B.1.1.7 variant, the SARS-CoV-2 B.1.351 variant and/or the SARS-CoV-2 B.1.617.2 variant; even more preferably, the term "SARS-CoV-2" further, i.e., in addition to wild-type SARS-CoV-2, includes the SARS-CoV-2 B.1.1.7 variant, the SARS-CoV-2 B.1.351 variant and the SARS-CoV-2 B.1.617.2 variant.

Preferably, when treating or preventing an infection with SARS-CoV2 in a human subject in need thereof by applying/using the composition comprising hypericin and/or pseudohypericin (preferably hypericin), said SARS-CoV2 comprises, in addition to wild-type SARS-CoV2, any one of the Alpha, Beta and/or Delta variant of SARS-CoV2.

More preferably, when treating or preventing an infection with SARS-CoV2 in a human subject in need thereof by applying/using the composition comprising hypericin and/or pseudohypericin (preferably hypericin), said SARS-CoV2 comprises, in addition to wild-type SARS-CoV-2, any one of the SARS-CoV-2 variant B.1.1.7, B.1.351 and/or B. 1.617.2.

Even more preferably, when treating or preventing an infection with SARS-CoV2 in a human subject in need thereof by applying/using the composition comprising hypericin and/or pseudohypericin (preferably hypericin), said SARS-CoV2 comprises in addition to wild-type SARS-CoV-2, the SARS-CoV-2 variant B.1.1.7.

Also even preferably, when treating or preventing an infection with SARS-CoV2 in a human subject in need thereof by applying/using the composition comprising hypericin and/or pseudohypericin (preferably hypericin), said SARS-CoV2 comprises in addition to wild-type SARS-CoV-2, the SARS-CoV-2 variant B.1.351.

Also even more preferably, when treating or preventing an infection with SARS-CoV2 in a human subject in need thereof by applying/using the composition comprising hypericin and/or pseudohypericin (preferably hypericin), said SARS-CoV2 comprises in addition to wild-type SARS-CoV-2, the SARS-CoV-2 variant B.1.617.2.

Also even more preferably, when treating or preventing an infection with SARS-CoV2 in a human subject in need thereof by applying/using the composition comprising hypericin and/or pseudohypericin (preferably hypericin), said SARS-CoV2 comprises in addition to wild-type SARS-CoV-2, the SARS-CoV-2 variant B.1.1.7 and B.1.351.

Also even more preferably, when treating or preventing an infection with SARS-CoV2 in a human subject in need thereof by applying/using the composition comprising hypericin and/or pseudohypericin (preferably hypericin), said SARS-CoV2 comprises in addition to wild-type SARS-CoV-2, the SARS-CoV-2 variant B.1.1.7 and B.1.617.2.

Also even more preferably, when treating or preventing an infection with SARS-CoV2 in a human subject in need thereof by applying/using the composition comprising hypericin and/or pseudohypericin (preferably hypericin), said SARS-CoV2 comprises in addition to wild-type SARS-CoV-2, the SARS-CoV-2 variant B.1.351 and B.1.617.2.

Particularly, preferably, when treating or preventing an infection with SARS-CoV2 in a human subject in need thereof by applying/using the composition comprising hypericin and/or pseudohypericin (preferably hypericin), said SARS-CoV2 comprises in addition to wild-type SARS-CoV-2, the SARS-CoV-2 variant B.1.1.7, B.1.351 and B.1.617.2.

As has surprisingly been shown in context with the present invention, compositions such as extracts comprising hypericin and/or pseudohypericin show beneficial effects in the treatment and prevention of SARS-CoV2 infections. Particularly, as has been found in context with the present invention, application of Hypericum perforatum (H.P.) extract, especially H.P. extract only, i.e. not comprising non-extracted H.P. (solid) plant material, such as aerial parts or substantial amounts of such aerial parts, has been shown effective in treating infections with SARS-CoV2.

Hence, it is preferred that compositions as disclosed herein do not comprise non-extracted H.P. (solid) plant material, such as aerial parts or substantial amounts of such aerial parts. It is also preferred that extracts of H.P. of the present invention do not comprise non-extracted H.P. (solid) plant material, such as aerial parts or substantial amounts of such aerial parts.

Even more surprisingly, it has been found that compositions described herein, such as extracts comprising hypericin and/or pseudohypericin, are active against SARS-CoV-2 variants B1.1.7, B.1.351 and/or B.617.2.

In context with the present invention, application of composition comprising hypericin and/or pseudohypericin (preferably hypericin) for use in a method for treating or preventing an infection with SARS-CoV2 in a human subject in need thereof can be in any suitable way, including, e.g., systemic administration or local administration.

In one embodiment of the present invention, the composition comprising hypericin and/or pseudohypericin (preferably hypericin) for use in a method for treating or preventing an infection with SARS-CoV2 in a human subject in need thereof is administered to the mucosa of said subject in need thereof.

Systemic administration may be conducted in any suitable way, e.g., oral, sublingual, enteral, parenteral (e.g., i.v., i.m., or s.c.).

In context with the present infection, local administration is preferably conducted to the mucosa of the human subject in need of being treated or prevented from SARS-CoV2 infection.

In context with the present invention, mucosa (where the composition comprising hypericin and/or pseudohypericin (preferably hypericin) as described and provided herein is applied to) may be selected from the group consisting of mucosa of the upper respiratory tract and mucosa from the lower respiratory tract, preferably the upper respiratory tract. In this context, for example and as one embodiment of the present invention, upper respiratory mucosa may be selected from the group consisting of nasal mucosa, oral mucosa (e.g., lining mucosa (incl. alveolar, buccal and labial mucosa), masticatory, and specialized mucosa), and pharyngeal mucosa (preferably oral and/or nasal mucosa). Furthermore, for example and as another embodiment of the present invention, lower respiratory mucosa may be selected from the group consisting of tracheal mucosa, bronchial mucosa and mucosa of the pulmonary alveoli (preferably bronchial and/or alveoli mucosa).

In one embodiment of the present invention, the hypericin and/or pseudohypericin comprised by the composition for use in a method for treating or preventing an infection with SARS-CoV2 in a human subject in need thereof as described and provided herein is comprised by an extract of *Hypericum perforatum* (H.P.).

In a more specific embodiment of the present invention, said composition comprising hypericin and/or pseudohypericin for use in a method for treating or preventing an infection with SARS-CoV2 in a human subject in need thereof as described and provided herein is an extract of H.P. In context with the present invention, such extract may be in accordance with state of the art pharmaceutical practices essentially, preferably completely void of (solid) plant material, such as aerial parts or substantial amounts of such aerial parts.

An "extract of Hypericum perforatum" or, as is equally referred to herein, an "extract of H.P." or an "extract of St. John's wort" is commonly known in the art, e.g as described in the following. Such an extract is preferably ready to be applied. Extracts of the plant Hypericum perforatum L. (popularly called St. John's wort) are widely used for treatment of patients with depressive symptoms. According to a meta analysis/systematic review, hypericum extracts are superior to placebo in patients with major depression, are similarly effective as standard antidepressants and have fewer side effects than standard antidepressants.

St. John's wort consists of the whole or cut, dried flowering tops of Hypericum perforatum L., plant family Hyperaceae, and are harvested during flowering time, mostly end of June.

The herbal material to be used for medicinal purposes has to comply in Europe to the quality specifications of the respective monograph of the Ph. Eur. 10.0, 1874 (2020), in particular Monograph Hyperici herbae extractum siccum quantificatum.

The herbal material contains not less than 0.08 % of total hypericins, expressed as hypericin and calculated with reference to the dried drug.

Additionally, European Pharmacopoeia monographs quantified St. John's wort dry extract (Hyperici herba extractum siccum quantificatum) (Ph. Eur. 10.0, 1874 (2020), in particular Monograph Hyperici herbae extractum siccum quantificatum), which defines the content of total hypericins, calculated as hypericin in the range of 0.10 to 0.30 %, the content of flavonoides, calculated as rutosid, to be > 6.0 % and the content of hyperforin to be less than 6.0 %.

The characteristic constituents of St. John's worth are naphthodianthrones and phloroglucinols. Naphthodianthrones (0.05-0.3 %), consisting mainly of hypericin and pseudohypericin 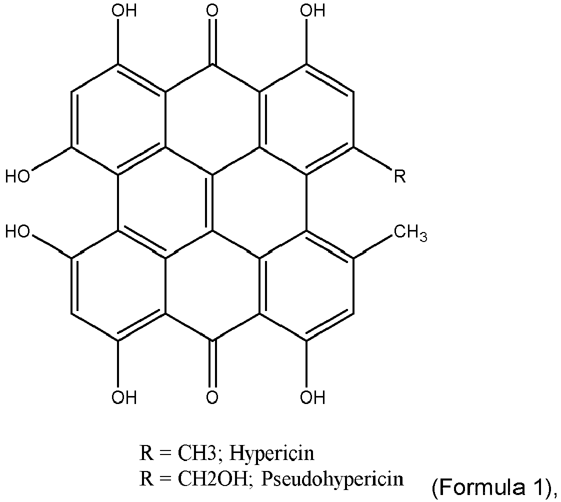 accumulate primarily in the flowers and buds; lower levels than 0.1% may result from harvesting of lower parts of the herb. Other naphthodianthrones present, and included in the term 'total hypericins', are the biosynthetic precursors protohypericin and protopseudohypericin (wich are transformed into hypericin and pseudohypericin respectively on exposure to light), and a small amount of cyclopseudohypericin. The principal phloroglucinols are hyperforin (2 - 4.5 %) 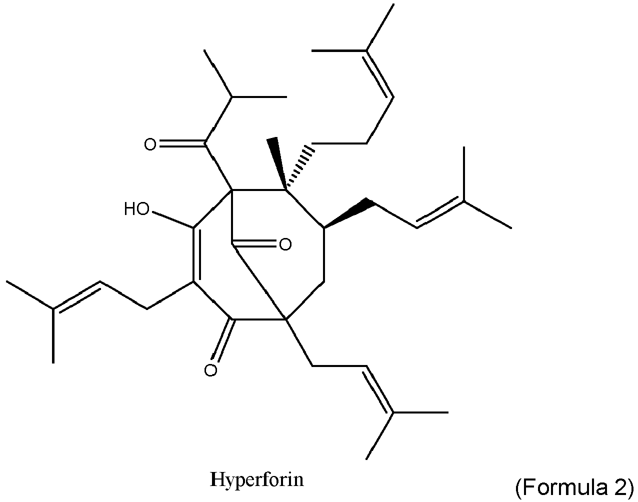 and adhyperforin (0.2 - 1.8%). Both compounds have limited stability and their oxidated derivatives are also present.

Quercetin glycosides (2 - 4%), including hyperoside, quercitrin, isoquercitrin and rutin, are the main flavonoids; a typical marker compound for St. John's wort is quercetin-3-O-glucuronid

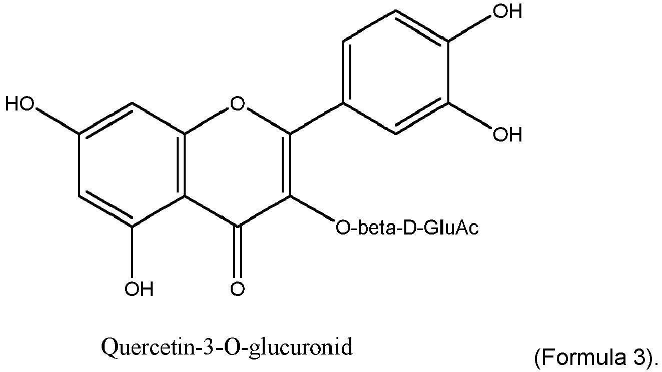

Also small amount of free quercetin can be detected. Higher amounts of flavan-3-ols (catechin, epicatechin) have been reported. Biflavonoids such as 13,ll8-biapigenin (0.1 - 0.5%) and 13', ll8-biapigenin (amentoflavone, 0.01-0.05%) occur exclusively in the flowers.

Other constituents include phenylpropanoids, such as chlorogenic acid and other caffeoylquinic and p-coumaroylquinic esters.

High contents of oligomeric and polymeric proanthocyanidins have been described (8 to 37 mg/g). In general the trimer cluster DP3 is the dominant proanthocyanidin (with about 40 % of the total PAC content) 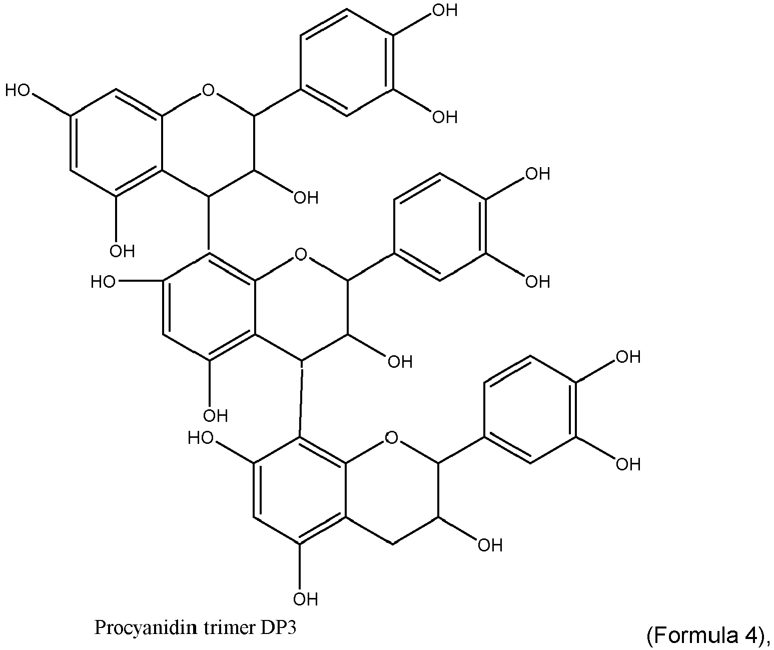 followed by DP 4 (about 15 %) and DP 5 (about 12 %). Concerning the structural features of these oligomer clusters the structures of procyanidins A2, B1, B2, B3, B5, B7 and C1 have been reported.

Additionally, trace amounts of xanthones such as 1,3,6,7-tetrahydroxyxanthone (0.0004 % in the leaves and stems). In addition, traces of volatile oil (0.1-0.25 %), containing mainly higher n-alkanes and monoterpenes has been described.

The antidepressant activity has been attributed in a pleiotropic manner to hyperforin and the naphtodianthrones. Because flavonol glycosides and the proanthocyanidins from the extract strongly interact with the solubility of naphthodianthrones in aqueous systems, these polyphenols have significant influence on the bioavailability of hypericin and can be considered to modify the clinical and functional efficacy of St. John's extracts.

In context with the present invention, such extract may be an excerpt with ethanol or methanol (preferably ethanol) as solvent, e.g. an H.P. extract according to Ph. Eur. 10.0, 1874 (2020), Monograph Hyperici herbae extractum siccum quantificatum, or US pharmacopoeia 41, 2018.

As such, the present invention also relates to an extract of Hypericum perforatum comprising hypericin and/or pseudohypericin for use in a method for treating or preventing an infection with SARS-CoV2 in a human subject in need thereof.

In one embodiment of the present invention, where hypericin and/or pseudohypericin is/are comprised by an extract of H.P. or where said composition comprising hypericin and/or pseudohypericin is an extract of H.P., such H.P. extract preferably does not comprise (solid) H.P. plant material, such as aerial parts or substantial amounts of such aerial parts.

In one embodiment of the present invention, such H.P. extract may be may be an excerpt with ethanol or methanol (preferably ethanol) as solvent, e.g. an H.P. extract according to Ph. Eur. 10.0, 1874 (2020), Monograph Hyperici herbae extractum siccum quantificatum, or US pharmacopoeia 41, 2018.

In another embodiment in this regard and in accordance with the present invention, such H.P. extract is not St. John's Wort from GAIA HERBS ^{®}, most preferably not St. John's Wort GAIA HERBS^{®} product described in Table 1 and/or Table 2 of Bajrai et al. (2021), loc. cit.; https://www.biorxiv.org/content/10.1101/2021.01.11.426295v1. In a more specific embodiment of the present invention, the composition comprising hypericin and/or pseudohypericin (preferably hypericin) for use in a method for treating or preventing an infection with SARS-CoV2 in a human subject in need thereof do not comprise St. John's Wort from GAIA HERBS ^{®}, most preferably do not comprise St. John's Wort GAIA HERBS^{®} product described in Table 1 and/or Table 2 of Bajrai et al. (2021), loc. cit.; https://www.biorxiv.org/content/10.1101/2021.01.11.426295v1.

In context with the present invention, in preferred embodiments, the H.P. extract comprising hypericin and/or pseudohypericin comprises hypericin and/or pseudohypericin at a concentration between about 0.1 w/w% and about 0.3 w/w%, preferably about 0.1 w/w%, about 0.2 w/w%, preferably about 0.2 w/w%, about 0.3 w/w%, more preferably about 0.3 w/w%.

In the context with the present invention, H.P. extract with a high content of hypericin, such as 0.2 w/w %, 0.3 w/w %, 0.4 w/w %, 0.5 w/w % or more than 0.5 w/w% is preferred.

Rather than an extract of H.P. comprising hypericin and/or pseudohypericin as described herein, compositions of the present invention comprise hypericin and/or pseudohypericin as such, i.e., as pure chemical compounds or substances. Both hypericin and pseudohypericin can be obtained from Hypericum perforatum, and are further purified and/or enriched, ideally up to purity, such that both hypericin and pseudohypericin are preferably pure chemical compounds. Thus, when both hypericin and pseudohypericin are referred to herein not in connection with an extract, it is meant that both hypericin and pseudohypericin are pure chemical compounds or substances.

In one embodiment of the present invention, the composition comprising hypericin and/or pseudohypericin (preferably hypericin) for use in a method for treating or preventing an infection with SARS-CoV2 in a human subject in need thereof as described and provided herein comprises or is a hypericin and/or pseudohypericin-comprising H.P extract as described herein.

In accordance with the present invention, the composition comprising hypericin and/or pseudohypericin (preferably hypericin) for use in a method for treating or preventing an infection with SARS-CoV2 in a human subject in need thereof as described and provided herein, and/or the H.P. extract comprising hypericin and/or pseudohypericin, may further comprise a solvent for hypericin and/or pseudohypericin, and/or a solvent for the H.P extract.

Suitable solvents for hypericin and/or pseudohypericin, as well as for the H.P extract are generally known in the art (cf., e.g., Ph. Eur. 10.0, 1874 (2020), Monograph Hyperici herbae extractum siccum quantificatum, or US pharmacopoeia 41, 2018) and comprise, e.g., pharmaceutically acceptable organic solvents such as ethanol or methanol, preferably ethanol.

In accordance with the present invention, the composition comprising hypericin and/or pseudohypericin (preferably hypericin) for use in a method for treating or preventing an infection with SARS-CoV2 in a human subject in need thereof as described and provided herein may be in any suitable form. In one embodiment of the present invention, it may be in liquid form (e.g., in form of a mouth wash, a gargle, a nose spray or drops, an ointment, e.g. nasal ointment, a gel, e.g. nasal gel, an inhalation spray or solution, or a nebulizer solution), in solid form (e.g., in form of a lozenge, or a tablet, a capsule, a pill), or in form of an injectable solution.

In a particular embodiment of the present invention, where the composition comprising hypericin and/or pseudohypericin (preferably hypericin) for use in a method for treating or preventing an infection with SARS-CoV2 in a human subject in need thereof as described and provided herein is administered to mucosa tissue of said human subject, preferably in form of a liquid solution (e.g., as a mouth wash, a gargle, a nose spray, a mouth or pharyngal spray, an ointment, a gel, an inhalation spray or solution, or a nebulizer solution), said composition may comprise an H.P extract in a concentration of preferably at least about 0.25 µg, about 0.5 µg, about 1 µg, about 1.5 µg, about 2.0 µg, about 2.5 µg H.P. extract per ml liquid solution, preferably at least about 3 µg, about 4 µg, about 5 µg, about 7.5 µg, about 10 µg, about 25 µg, about 30 µg, about 35 µg, about 40 µg, or about 45 µg H.P. extract per ml liquid solution.

The above being said, it is nevertheless also envisaged that in a further particular embodiment of the present invention, where the composition comprising hypericin and/or pseudohypericin (preferably hypericin) for use in a method for treating or preventing an infection with SARS-CoV2 in a human subject in need thereof as described and provided herein is administered to mucosa tissue of said human subject, preferably in form of a liquid solution (e.g., as a mouth wash, a gargle, a nose spray, a mouth or pharyngal spray, an ointment, a gel, an inhalation spray or solution, or a nebulizer solution), said composition may comprise an H.P. extract in a concentration of preferably at least about 50 µg H.P. extract per ml liquid solution, preferably at least about 60 µg, about 70 µg, about 80 µg, about 90 µg, about 100 µg, about 110 µg, about 120 µg, about 130 µg, about 140 µg, or about 150 µg H.P. extract per ml liquid solution.

In a particular embodiment of the present invention, where the composition comprising hypericin and/or pseudohypericin (preferably hypericin) for use in a method for treating or preventing an infection with SARS-CoV2 in a human subject in need thereof as described and provided herein is administered to mucosa tissue of said human subject, preferably in form of a liquid solution (e.g., as a mouth wash, a gargle, a nose spray, a mouth or pharyngal spray, an ointment, a gel, an inhalation spray or solution, or a nebulizer solution), said composition may comprise hypericin and/or pseudohypericin in a concentration of preferably about 0.25 ng/ml, about 0.5 ng/ml, about 1 ng/ml, about 2 ng/ml per ml liquid solution, preferably at least about 5 ng/ml, preferably at least about 7.5 ng/ml, preferably at least about 10 ng/ml, preferably at least about 25 ng/ml, preferably at least about 50 ng/ml, preferably at least about 100 ng/ml, preferably at least about 200 ng/ml, preferably at least about 300 ng/ml, preferably at least about 400 ng/ml, preferably at least about 500 ng/ml, preferably at least about 600 ng/ml, preferably at least about 700 ng/ml, preferably at least about 800 ng/ml, preferably at least about 900 ng/ml, or preferably at least about 1000 ng/ml, per ml liquid solution.

In a particular embodiment of the present invention, where the composition comprising hypericin and/or pseudohypericin (preferably hypericin) for use in a method for treating or preventing an infection with SARS-CoV2 in a human subject in need thereof as described and provided herein is administered parenterally, such as i.v., i.m., or s.c. to said human subject, preferably in form of a liquid solution (e.g., as injectable solution), said composition may comprise hypericin and/or pseudohypericin in a concentration of preferably about 0.25 ng/ml, about 0.5 ng/ml, about 1 ng/ml, about 2 ng/ml per ml liquid solution, preferably at least about 5 ng/ml, preferably at least about 7.5 ng/ml, preferably at least about 10 ng/ml, preferably at least about 25 ng/ml, preferably at least about 50 ng/ml, preferably at least about 100 ng/ml, preferably at least about 200 ng/ml, preferably at least about 300 ng/ml, preferably at least about 400 ng/ml, preferably at least about 500 ng/ml, preferably at least about 600 ng/ml, preferably at least about 700 ng/ml, preferably at least about 800 ng/ml, preferably at least about 900 ng/ml, or preferably at least about 1000 ng/ml, per ml liquid solution

In a further embodiment of the present invention, the total daily dose of the composition, when, e.g. administered enterally, e.g., in the form of or a tablet, a capsule, or a pill, comprising hypericin and/or pseudohypericin (preferably hypericin) for use in a method for treating or preventing an infection with SARS-CoV2 in a human subject in need thereof as described and provided herein administered to said subject does preferably not exceed about 86400 µg hypericin and/or pseudohypericin in total, preferably does not exceed about 43200 µg hypericin and/or pseudohypericin in total, preferably does not exceed about 21600 µg hypericin and/or pseudohypericin in total, preferably does not exceed about 10800 µg hypericin and/or pseudohypericin in total, preferably does not exceed about 8100 µg hypericin and/or pseudohypericin in total, preferably does not exceed about 7200 µg hypericin and/or pseudohypericin in total, preferably does not exceed about 5400 µg hypericin and/or pseudohypericin in total, preferably does not exceed about 3600 µg hypericin and/or pseudohypericin in total, preferably does not exceed about 2700 µg hypericin and/or pseudohypericin in total, preferably does not exceed about 1800 µg hypericin and/or pseudohypericin in total, preferably does not exceed about 900 µg hypericin and/or pseudohypericin in total, preferably does not exceed about 600 µg hypericin and/or pseudohypericin in total, or preferably does not exceed about 300 µg hypericin and/or pseudohypericin in total. When referred to herein to "in total" this means preferably "in total per day".

In this context, in a specific embodiment of the present invention where the composition comprising hypericin and/or pseudohypericin (preferably hypericin) for use in a method for treating or preventing an infection with SARS-CoV2 in a human subject in need thereof as described and provided herein is administered to said human subject in solid form (e.g., in form of a lozenge, or a tablet, a capsule, a pill), the total daily dose of composition comprising hypericin and/or pseudohypericin (preferably hypericin) for use in a method for treating or preventing an infection with SARS-CoV2 in a human subject in need thereof as described and provided herein administered to said subject does preferably not exceed about 86400 µg hypericin and/or pseudohypericin in total, preferably does not exceed about 43200 µg hypericin and/or pseudohypericin in total, preferably does not exceed about 21600 µg hypericin and/or pseudohypericin in total, preferably does not exceed about 10800 µg hypericin and pseudohypericin in total, preferably does not exceed about 8100 µg hypericin and/or pseudohypericin in total, preferably does not exceed about 7200 µg hypericin and/or pseudohypericin in total, preferably does not exceed about 5400 µg hypericin and/or pseudohypericin in total, preferably does not exceed about 3600 µg hypericin and/or pseudohypericin in total, preferably does not exceed about 2700 µg hypericin and/or pseudohypericin in total, preferably does not exceed about 1800 µg hypericin and/or pseudohypericin in total, preferably does not exceed about 900 µg hypericin and/or pseudohypericin in total, preferably does not exceed about 600 µg hypericin and/or pseudohypericin in total, or preferably does not exceed about 300 µg hypericin and/or pseudohypericin in total. When referred to herein to "in total" this means preferably "in total per day".

In a further embodiment of the present invention, the total daily dose of the H.P. extract comprised by the composition as described and provided herein administered to said subject preferably does not exceed about 28800 mg, preferably does not exceed about 14400 mg, preferably does not exceed about 7200 mg preferably does not exceed about 3600 mg, preferably does not exceed about 2700 mg, preferably does not exceed about 1800 mg, preferably does not exceed about 900 mg, preferably does not exceed about 600 mg or preferably does not exceed about 300 mg.

In this context, in a specific embodiment of the present invention where the composition comprising hypericin and/or pseudohypericin (preferably hypericin) for use in a method for treating or preventing an infection with SARS-CoV2 in a human subject in need thereof as described and provided herein is administered to said human subject in solid form (e.g., in form of a lozenge, or a tablet, a capsule, a pill), the total daily dose of the H.P. extract comprised by the composition as described and provided herein administered to said subject preferably does not exceed about 28800 mg, preferably does not exceed about 14400 mg, preferably does not exceed about 7200 mg preferably does not exceed about 3600 mg, preferably does not exceed about 2700 mg, preferably does not exceed about 1800 mg, preferably does not exceed about 900 mg, preferably does not exceed about 600 mg or preferably does not exceed about 300 mg.

When applying the composition comprising hypericin and/or pseudohypericin (preferably hypericin) for use in a method for treating or preventing an infection with SARS-CoV2 in a human subject in need thereof as described and provided herein enterally (preferably comprised by an H.P. extract in solid form, e.g., in form of a lozenge, or a tablet, a capsule, a pill), the hypericin and/or pseudohypericin will be absorbed and enter the plasma, thereby resulting in a certain plasma concentration.

Literature reports for a single dose regimen:
300, 900, and 1800 mg oral extract yielded hypericin peak plasma levels of 1.5, 7.5, and 14.2 ng/ml (Biber et al 1998, Pharmacopsychiatry. 1998 Jun; 31 Suppl 1:36-43).
250, 750, and 1500 µg hypericin in orally administered extract yielded mean peak plasma levels of 1.3, 7.2, and 16.6 ng/ml hypericin (Kerb et al. (1996), Antimicrobial Agents and Chemotherapy 40(9): 2087-2093).
115 µg hypericin in intravenously administered extract yielded initial peak plasma levels of 29.5 ng/ml hypericin (Kerb et al, loc. cit.).

Literature reports for multiple dose regimen:
0.05 mg of hypericin per kg of body and 0.1 mg of hypericin per kg of body orally administered daily for 8 weeks yielded mean minimum plasma levels of 15.4 and 41.7 ng/ml and mean maximum plasma levels of 28.1 and 79.2 ng/ml (Jacobson et al. (2001), Antimicrobial Agents and Chemotherapy, 45(2): 517-524).

Thus, hypericin plasma levels in the range from 0 to 75 ng/ml may be well achieved by administering appropriate oral or parenteral preparations of hypericum extract or (pure) hypericin. Due to the long elimination half time (T1/2) which has been reported dose dependently with 24.5 h up to 48 h (Kerb et al 1996, loc. cit.) and 24.8 and 26.6 h (Biber et al 1998, loc. cit.), it can be assumed that maximum daily peak plasma levels during longterm treatment may due to cumulation amount to the 2 - 3-fold of those seen with single dose regimen and trough plasma levels will not fall off more than 50%.

The following table provides an overview how maximum and minimum plasma levels may present dependent on the hypericin-%age of the extract dose orally administered (0.1% - 0.3%).

| extract | hypericin 0.1% | hypericin 0.2% | hypericin 0.3% |
|---|---|---|---|
| 300 mg | 300 µg | 600 µg | 900 µg |
| expected plasma conc. | 0.88-1.66 ng/ml | 1.77-3.33 ng/ml | 2.66-5 ng/ml |

| extract | hypericin 0.1% | hypericin 0.2% | hypericin 0.3% |
|---|---|---|---|
| 900 | 900 µg | 1800 µg | 2700 µg |
| expected plasma conc. | 2.66-5 ng/ml | 5.33-10 ng/ml | 8-15 ng/ml |

| extract | hypericin 0.1% | hypericin 0.2% | hypericin 0.3% |
|---|---|---|---|
| 1800 | 1800 µg | 3600 µg | 5400 µg |
| expected plasma conc. | 5.33-10 ng/ml | 10.66-20 ng/ml | 16-30 ng/ml |

| extract | hypericin 0.1% | hypericin 0.2% | |
|---|---|---|---|
| 2700 | 2700 µg | 5400 µg | 8100 µg |
| expected plasma conc. | 8-15 ng/ml | 16-30 ng/ml | 24-45 ng/ml |

| extract | hypericin 0.1% | hypericin 0.2% | hypericin 0.3% |
|---|---|---|---|
| 3600 | 3600 µg | 7200 µg | 10800 µg |
| expected plasma conc. | 10.66-20 ng/ml | 21.33-40 ng/ml | 32-60 ng/ml |

These values fit with plasma concentrations reported in the literature, e.g. Schulz et al. (2005), Arzneimittelforschung 55(1):15-22; Fox et al., (2001), Cancer Chemother Pharmacol. 47(1):41-44; Kerb et al. (1996), Antimicrobial Agents and Chemotherapy 40(9): 2087-2093.

Since the H.P. extract of the present invention is not toxic at concentrations where the H.P. extract used by Bajrai (loc. cit.), i.e. 1.56 µ/ml, showed toxicity, it is self-speaking that in the context of the present invention higher amount of extract and, thus, higher amount of hypericin can be applied and/or administered.

Accordingly, when applying the composition comprising hypericin and/or pseudohypericin (preferably hypericin) for use in a method for treating or preventing an infection with SARS-CoV2 in a human subject in need thereof as described and provided herein enterally (preferably comprised by an H.P. extract in solid form, e.g., in form of a lozenge, or a tablet, a capsule, a pill), the hypericin and/or pseudohypericin will be absorbed and enter the plasma and, thus, it is preferred that either H.P. extract or hypericin and/or pseudohypericin is administered in an amount such that the plasma concentration of hypericin is more than 0.25 ng/ml, 0.5 ng/ml, 1 ng/ml, 2 ng/ml, 3 ng/ml, such as 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74 or 75 ng/ml. Alternatively, plasma concentration of hypericin may be between 3-60 ng/ml, such as 3-5 ng/ml, 5-10 ng/ml, 8-15 ng/ml, 10-20 ng/ml, 16-30 ng/ml, 20-40 ng/ml, 24-45 ng/ml or 30-60 ng/ml.

As is illustrated in the Table above, the skilled person is readily in a position to determine the amount of H.P. extract (comprising hypericin and/or pseudohypericin) required to reach a desired plasma concentration of hypericin as described herein.

In a further embodiment of the present invention, the composition comprising hypericin and/or pseudohypericin (preferably hypericin) for use in a method for treating or preventing an infection with SARS-CoV2 in a human subject in need thereof as described and provided herein comprises hyperforin at a concentration of not more preferably of not more than 5 µg/ml, 2 µg/ml, more preferably of not more than 1 µg/ml, more preferably of not more than 0.5 µg/ml, more preferably of not more than 0.1 µg/ml, more preferably of not more than 0.05 µg/ml, more preferably of not more than 0.01 µg/ml, more preferably of not more than 0.001 µg/ml, and is most preferably essentially void of hyperforin.

In a further embodiment of the present invention, the composition comprising hypericin and/or pseudohypericin (preferably hypericin) for use in a method for treating or preventing an infection with SARS-CoV2 in a human subject in need thereof as described and provided herein is a pharmaceutical composition. In accordance with the present invention, such pharmaceutical composition may further comprise suitable pharmaceutically acceptable carriers and galenic ingredients as generally known in the art (cf., e.g., European Pharmacopoeia (Ph.Eur.), 10^{th} edition 2020, or US pharmacopoeia 41, 2018).

The present invention further relates to a mouth wash, gargle solution, nose spray, inhalant, lozenge, chewing gum comprising hypericin and/or pseudohypericin, and/or an H.P. extract comprising hypericin and/or pseudohypericin as described herein.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% or 2% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of may be replaced with either of the other two terms.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### Figures

The Figures show:

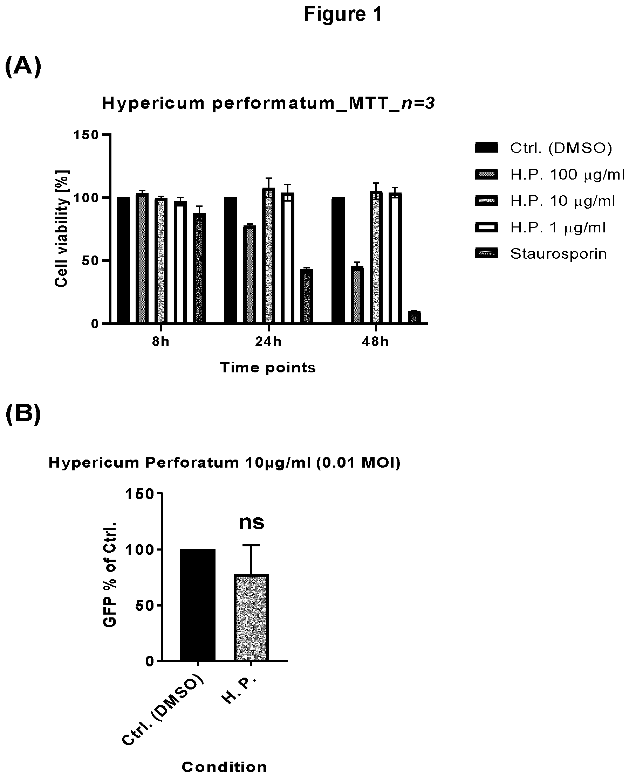

**Figure 1**: Hypericum perforatum (H.P.) testing against VSV-ΔG SARS CoV-2 S pseudovirus. **(A)** MTT assay of H.P. (100, 10, 1µg/ml) on Vero cells. Cells were seeded for overnight then cells were incubated for the indicated time points with new media containing DMSO (as a solvent control) or different concentrations of H.P. Values were expressed as a percentage of mean control (DMSO). **(B)** The impact of H.P. on the VSV-ΔG- SARS CoV-2 S pseudovirus. Vero cells were seeded overnight, then treated with DMSO or 10µg/ml H.P. for 1h at 37°c. Meanwhile, the pseudovirus was incubated with either DMSO or 10µg/ml H.P. for 1h at room temperature. The cells were incubated with the virus for 1h at 37°c for adsorption, followed by 1x DMEM 10% FCS washing, and finally 100µl of new DMEM 10% FCS for 16-18h. Results were expressed as a percentage of GFP positive cells of the control (DMSO), where ns means non-significant statistical results of student T test (at least three independent experiments).

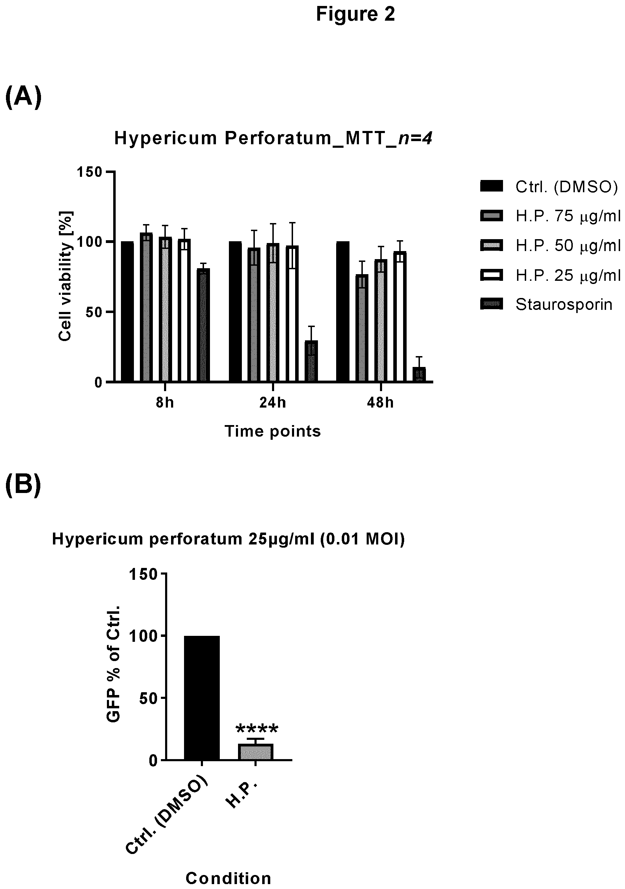

**Figure 2****:** Hypericum perforatum (H.P.) testing against VSV-ΔG SARS CoV-2 S pseudovirus. (A) MTT assay of H.P. (75, 50, 25µg/ml) on Vero cells. Cells were seeded for overnight and then incubated for the indicated time points with new media that contains DMSO (as a solvent control) or different concentrations of H.P. Values were expressed as a percentage of mean control (DMSO). (B) The impact of H.P. on the VSV-ΔG SARS CoV-2 S pseudovirus. Vero cells were seeded overnight, then treated with DMSO (as a control) or 25µg/ml H.P. for 1h at 37oc. Meanwhile, the pseudovirus was incubated with either DMSO or 25µg/ml H.P. for 1h at room temperature. The cells were incubated with the virus for 1h at 37oc for adsorption, followed by 1x DMEM 10% FCS washing, and finally 100µl of new DMEM 10% FCS. Results were expressed as a percentage of GFP positive cells of the control (DMSO), where **** means significant results of student T test (p value <0.0001) (at least three independent experiments).

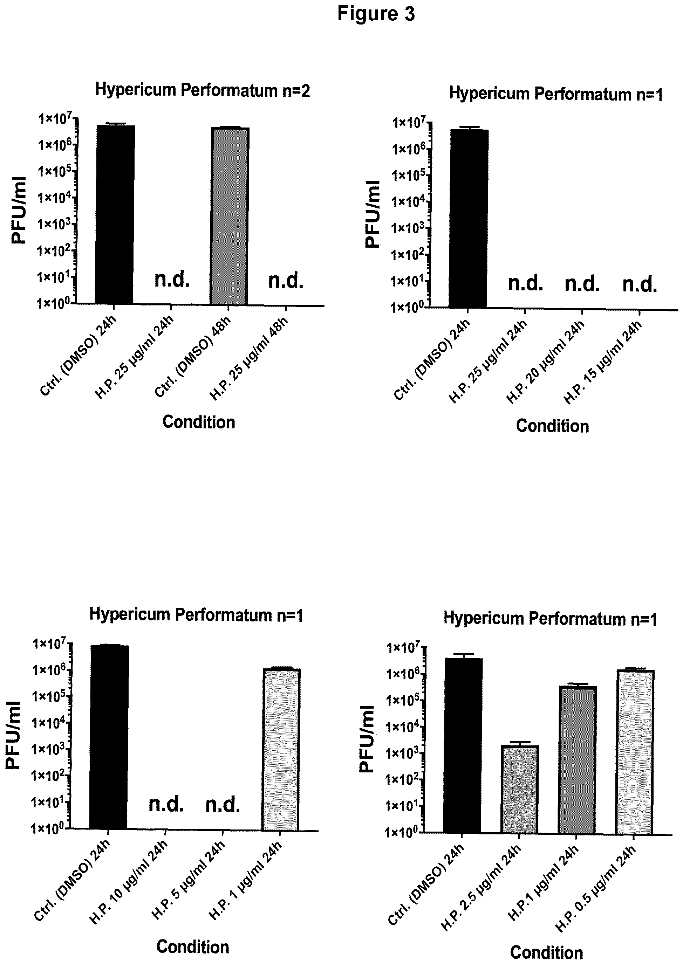

**Figure 3****:** Hypericum perforatum (H.P.) testing against SARS CoV-2. Vero cells were seed overnight. On the next day, cells were incubated with SARS CoV-2 infection media (2% FCS) containing DMSO (as a solvent control) or H.P. (indicated concentrations). Meanwhile, the SARS CoV-2 was incubated in PBS infection mix (MOI 0.05) containing DMSO or H.P. for 1 hour at room temperature. Following infection of cells lasted for 1h. After infection, cells were incubated with new infection DMEM (2% FCS) containing DMSO (as solvent control) or H.P. (different concentrations tested) for 24h or 48h in total. Supernatants were subjected to standard plaque assay and titers were expressed as plaque forming units per ml (PFU/ ml). n.d. means non-detectable.

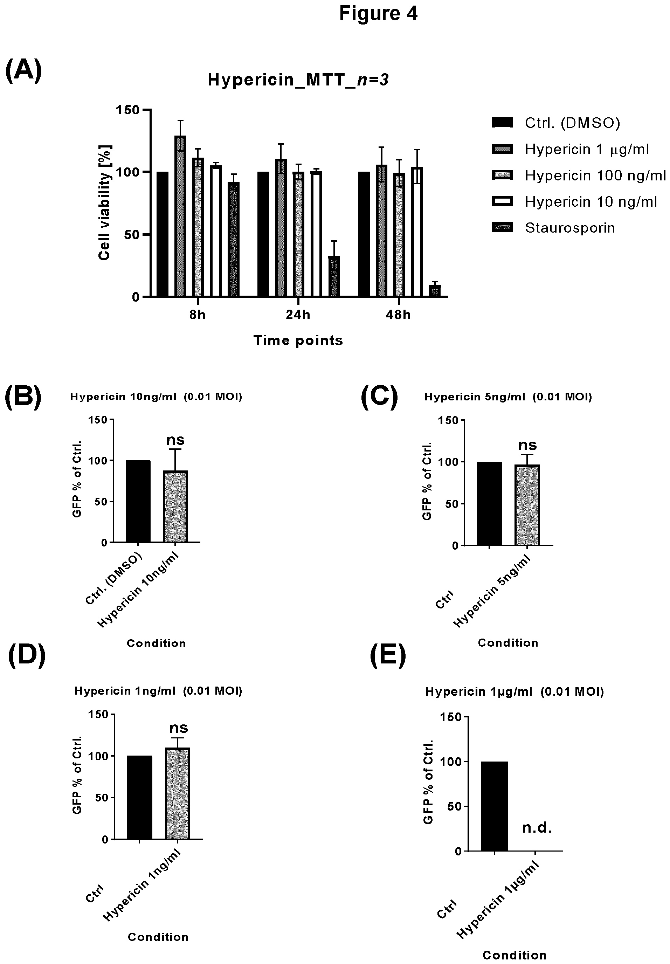

**Figure 4****:** Hypericin (an active component in Hypericum perforatum) testing against VSV-ΔG SARS CoV-2 S pseudovirus. **(A)** MTT assay of Hypericin (1µg/ml, 100ng/ml, 10ng/ml) on Vero cells. Cells were seeded for overnight and then cells were incubated for the indicated time points with new media that contains DMSO (as a solvent control) or different concentrations of Hypericin. Values were expressed as a percentage of mean solvent control (DMSO). **(B-E)** The impact of Hypericin on the VSV-ΔG SARS CoV-2 S pseudovirus. Vero cells were seeded overnight and then treated with DMSO (as a solvent control) or (B) 10ng/ml (C) 5ng/ml (D) 1 ng/ml or E) 1µg/ml of Hypericin for 1h at 37°c. Meanwhile, the pseudovirus was incubated with either DMSO or same corresponding concentrations of Hypericin for 1h at room temperature. Afterwards the cells were incubated with the virus for 1h at 37°c for adsorption, followed by 1x DMEM 10% FCS washing and finally 100µl of new DMEM 10% FCS for nearly 16-18 hours. Results were expressed as a percentage of GFP positive cells of the solvent control (DMSO), where ns means non-significant statistical results of student T test and n.d. means non-detectable (at least three independent experiments).

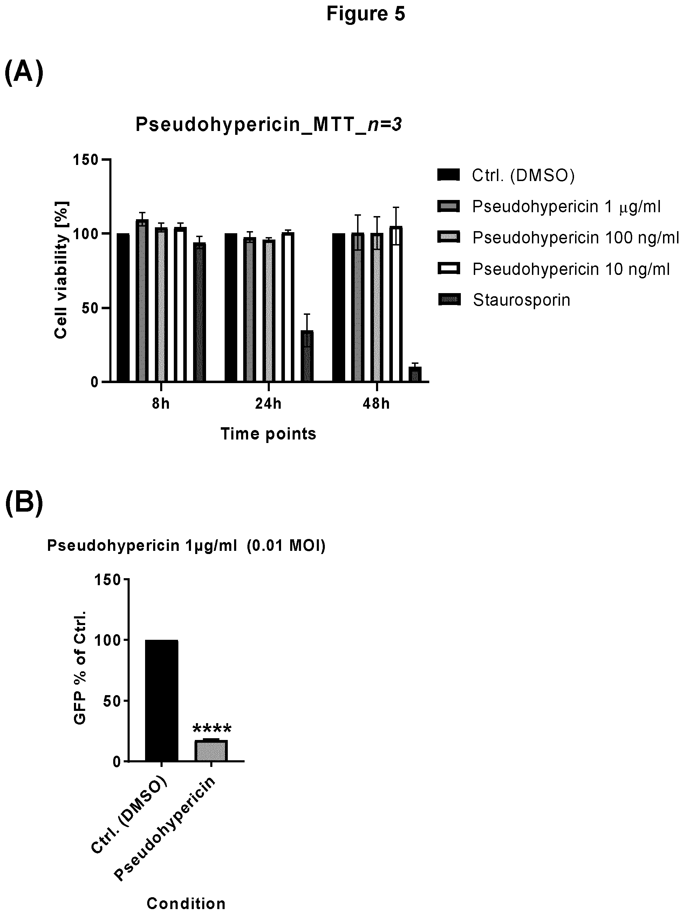

**Figure 5****:** Pseudohypericin (one active component in Hypericum perforatum) testing against VSV-ΔG SARS CoV-2 S pseudovirus. **(A)** MTT assay of Pseudohypericin (1µg/ml, 100ng/ml, 10ng/ml) on Vero cells. Cells were seeded for overnight and then cells were incubated for the indicated time points with new media that contains DMSO (as a control) or different concentrations of Pseudohypericin. Values were expressed as a percentage of mean control (DMSO). **(B)** The impact of Pseudohypericin on the VSV-ΔG SARS CoV-2 S pseudovirus. Vero cells were seeded overnight, and then treated with DMSO (as a control) or 1µg/ml Pseudohypericin for 1h at 37°c. Meanwhile, the pseudovirus was incubated with either DMSO or 1µg/ml pseudohypericin for 1h at room temperature. Afterwards, the cells were incubated with the virus for 1h at 37°c for adsorption, followed by 1x DMEM 10% FCS washing, and finally 100µl of new DMEM 10% FCS for nearly 16-18 hours. Results were expressed as a percentage of GFP positive cells of the control (DMSO), where **** means significant statistical results of student T test (p value <0.0001) (at least three independent experiments).

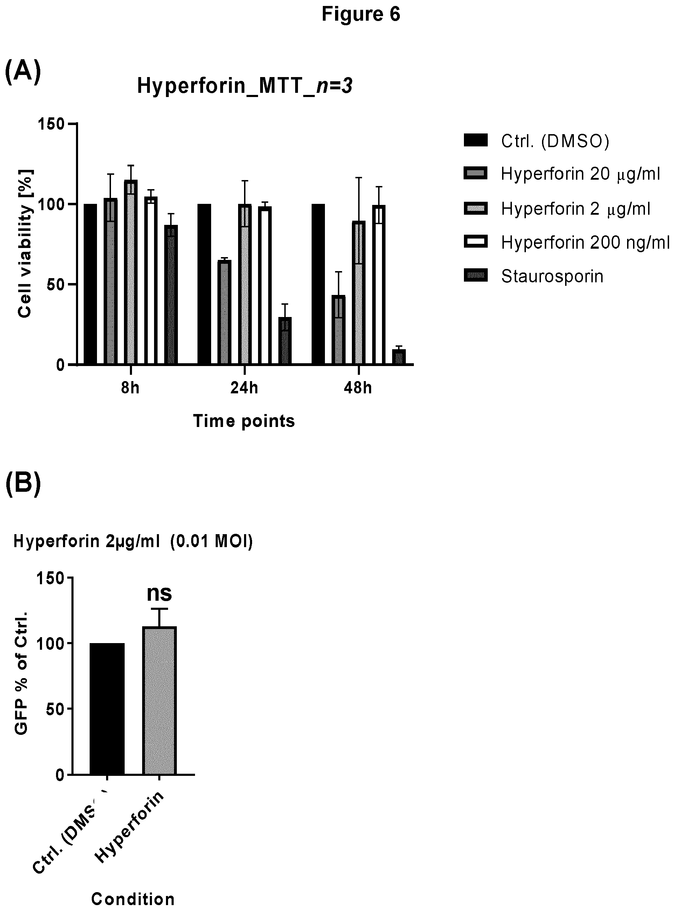

**Figure 6****:** Hyperforin (one active component in Hypericum perforatum) testing against VSV-ΔG SARS CoV-2 S pseudovirus. **(A)** MTT assay of Hyperforin (20, 2µg/ml, 200ng/ml) on Vero cells. Cells were seeded for overnight and then cells were incubated for the indicated time points with new media that contains DMSO (as a control) or different concentrations of Hyperforin. Values were expressed as a percentage of mean control (DMSO). **(B)** The impact of Hyperforin on the VSV-ΔG SARS CoV-2 S pseudovirus. Vero cells were seeded overnight, and then treated with DMSO (as a control) or 2µg/ml Hyperforin for 1h at 37°c. Meanwhile, the pseudovirus was incubated with either DMSO or 2µg/ml Hyperforin for 1h at room temperature. Afterwards, the cells were incubated with the virus for 1h at 37°c for adsorption, followed by 1x DMEM 10% FCS washing, and finally 100µl of new DMEM 10% FCS for nearly 16-18 hours. Results were expressed as a percentage of GFP positive cells of the control (DMSO), where ns means non-significant results of student T test (at least three independent experiments).

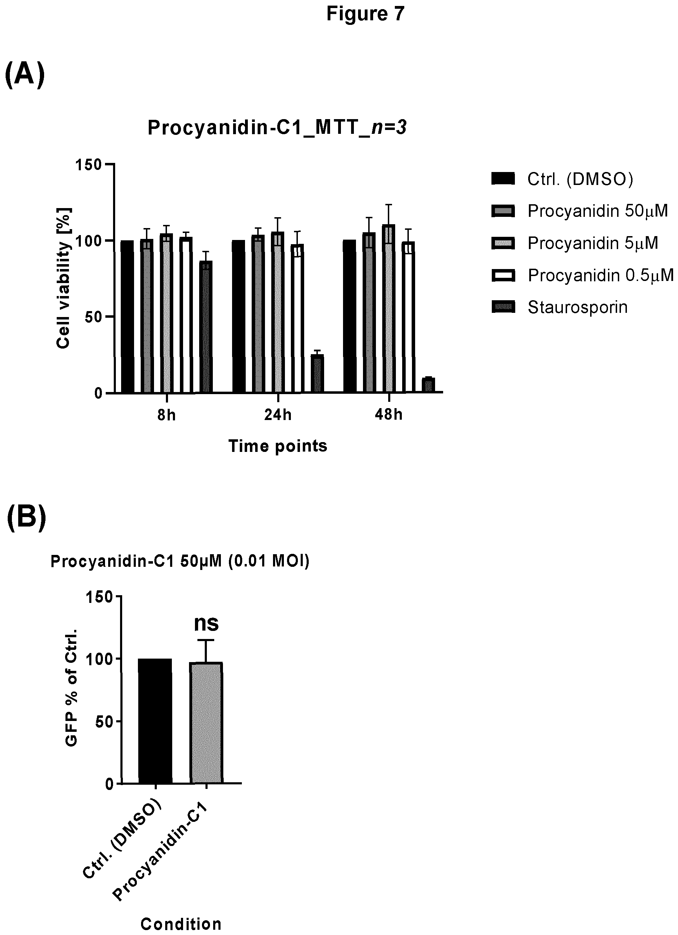

**Figure 7****:** Procyanidin C1 testing against VSV-ΔG SARS CoV-2 S pseudovirus. (A) MTT assay of Procyanidin C1 (50, 5, 0.5µM) on Vero cells. Cells were seeded for overnight and then cells were incubated for the indicated time points with new media that contains DMSO (as a control) or different concentrations of Procyanidin C1. Values were expressed as a percentage of mean control (DMSO). **(B)** The impact of Procyanidin C1 on the VSV-ΔG SARS CoV-2 S pseudovirus. Vero cells were seeded overnight, and then treated with DMSO (as a control) or 50µM Procyanidin C1 for 1h at 37°c. Meanwhile, the pseudovirus was incubated with either DMSO or 50µM Procyanidin C1 for 1h at room temperature. Afterwards, the cells were incubated with the virus for 1h at 37°c for adsorption, followed by 1x DMEM 10% FCS washing, and finally 100µl of new DMEM 10% FCS for nearly 16-18 hours. Results were expressed as a percentage of GFP positive cells of the control (DMSO), where ns means non-significant results of student T test (at least three independent experiments).

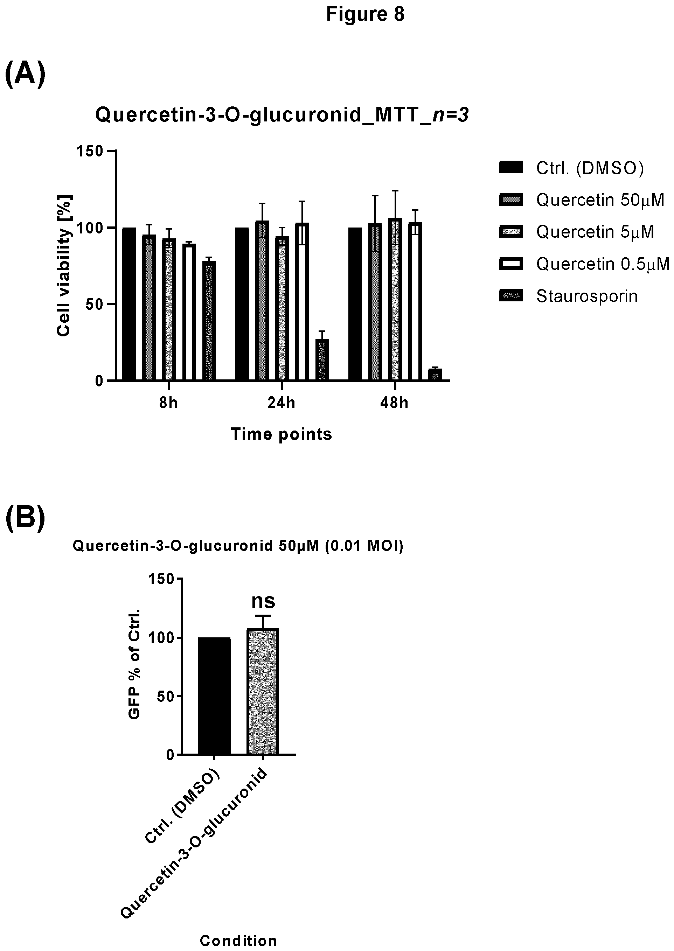

**Figure 8****:** Quercetin-3-O-glucuronid testing against VSV- ΔG SARS CoV-2 S pseudovirus. **(A)** MTT assay of Quercetin (50, 5, 0.5µM) on Vero cells. Cells were seeded for overnight and then cells were incubated for the indicated time points with new media that contains DMSO (as a control) or different concentrations of Quercetin. Values were expressed as a percentage of mean control (DMSO). **(B)** The impact of Quercetin on the VSV-ΔG SARS CoV-2 S pseudovirus. Vero cells were seeded overnight, and then treated with DMSO (as a control) or 50µM Quercetin for 1h at 37°c. Meanwhile, the pseudovirus was incubated with either DMSO or 50µM Quercetin for 1h at room temperature. Afterwards, the cells were incubated the virus for 1h at 37°c for adsorption, followed by 1x DMEM 10% FCS washing, and finally 100µl of new DMEM 10% FCS for nearly 16-18 hours. Results were expressed as a percentage of GFP positive cells of the control (DMSO), where ns means non-significant results of student T test (at least three independent experiments).
**Figure 9****:** The impact of Hypericum perforatum or Hypericin on SARS-CoV2 Variants of Concern (short: VOC). Vero cells were seeded overnight, then preincubated with IDMEM-2%FCS, which include solvent control (DMSO) or final concentrations of Hypericum perforatum (H.P. 15µg/ml) or Hypericin (HY. 10ng/ml) at 37°C/1h. Meanwhile, the SARS-CoV-2 VOC was incubated with I-PBS mix that includes solvent control (DMSO) or same final concentrations of Hypericum or Hypericin at room temperature/1h. After cell and virus pre-treatment, cells were infected at 37°C for 1h. After the infection, cells were washed with normal PBS and further incubated with I-DMEM including solvent control (DMSO) or same concentrations of Hypericum or Hypericin. 24h after infection, supernatants were collected, and standard plaque assay was performed to obtain PFU/ml values. Shown are the mean and SD of at least three independent experiments.

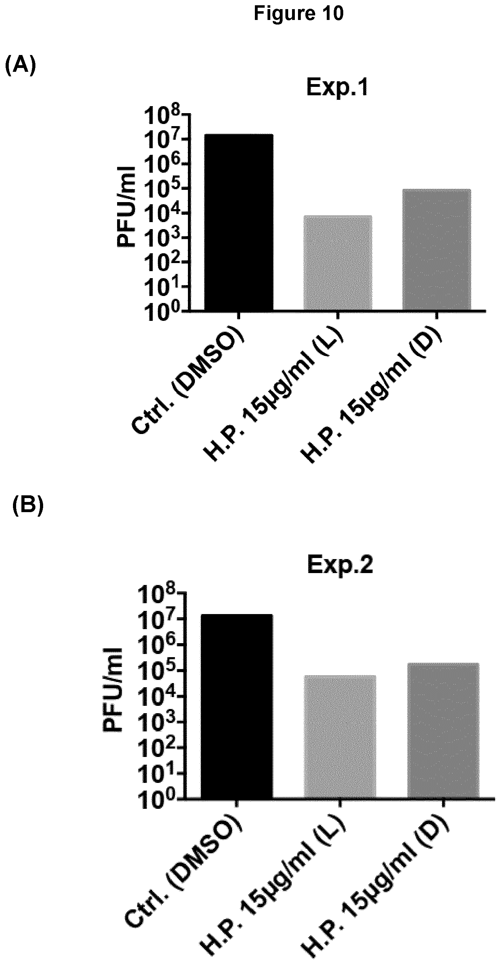

**Figure 10****:** Virus reduction (measured by PFU/ml values) when hypericum perforatum (H.P.) 15µg/ml was applied to infected cells and the cells were left in the dark (D) or in the light (L). Two experiments have been performed (see experiment 1 **(A)** and experiment 2 **(B)**).

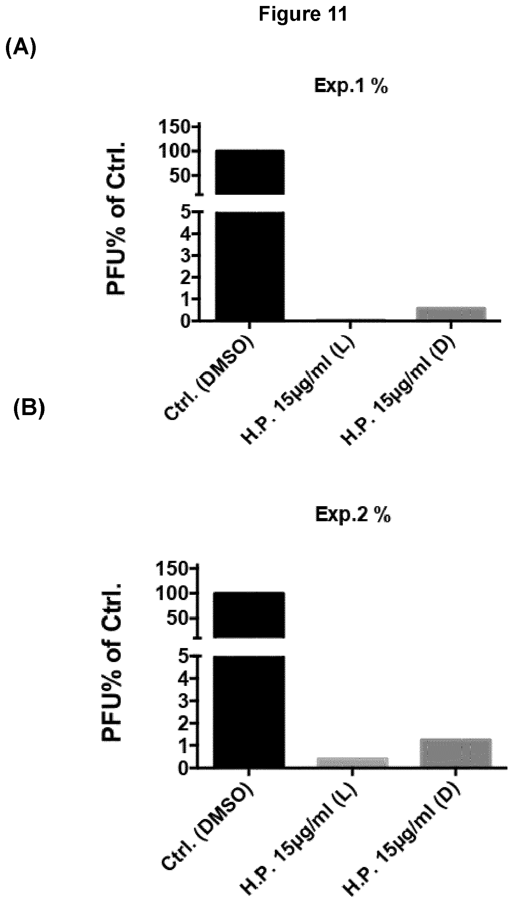

**Figure 11****:** Virus reduction (measured by PFU% of the DMSO control) when hypericum perforatum (H.P.) 15µg/ml was applied to infected cells and the cells were left in the dark (D) or in the light (L). Two experiments have been performed (see experiment 1 **(A)** and experiment 2 **(B)**).

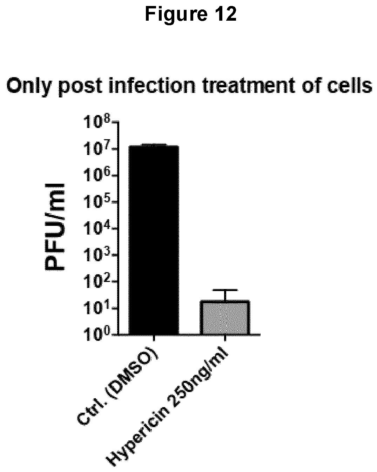

**Figure 12****:** Virus reduction (measured by PFU/ml values) when hypericin 250ng/ml was applied to infected cells, (i.e. without pre-treatment of the virus).

### Examples

The present invention is further illustrated by the following examples. Yet, the examples and specific embodiments described therein must not be construed as limiting the invention to such specific embodiments.

### Protocols

### 1. MTT-Assay:

- On day 1: Vero cells (1 × 10⁴ cells/well) were seeded in 100 µl media (4 per treatment) and incubate at 37 °C.
- On day 2: Preferred drug concentrations were diluted in DMEM 10% FCS. Also, controls (solvent, Staurosporin (1 µM)) were prepared. Supernatants of cells were removed and substances applied for 8h, 24h and 48h at 37°C.
- On day 2-4: fresh MTT [3-(4,5-Dimethylthiazol-2yl)-2,5-diphenyl-tetrazolium-bromid] was diluted in PBS to a final concentration of 5 mg/ml and 25 µl/well of MTT-solution were directly added to the wells and incubated for 1 h at 37°C.
- MTT supernatant was removed and cells were lysed by 50 µl/well of 100 % DMSO and incubated for 1-5 minutes at room temperature.
- Measurements were done at 562 nm in a photometer and % cell survival were calculated as = 100 × OD (sample) / OD (control).

### 2. Production of VSV Pseudovirus (VSV-ΔG - SARS CoV-2 S spike d21):

- On day 1, 293T cells were seeded overnight (5×10⁶) in DMEM 10% FCS (10ml total volume) in a poly-L-lysine coated 10cm dish.
- On day 2, the mutant SARS CoV2-d21 expression plasmid is transfected into 293T cells using OptiMEM and TransIT transfection reagent mixture in a new DMEM 10% FCS
- On day 3, 293T cells got infected with VSV-ΔG +G virus for 1h/37°c (DMEM-10% FCS infection mix). Cells got washed with DMEM 10% FCS, incubated with anti VSV G protein antibody for 30 minutes/37°c (to neutralize potentially remaining VSV-ΔG +G viruses), washed twice with DMEM 10% FCS, and incubated for 18-22h/37°c with fresh DMEM 10% FCS.
- On day 4, supernatants were collected, briefly centrifuged to remove cellular debris, introduced to a centrifugation 100KD column for concentration, and finally titrated in Vero cells (cells were visualized under fluorescent microscope (GFP positive cells are green in color)) to get a count of GFP positive cells used for calculation of virus titer in foci forming units/ml (FFU/ml) in both solvent control and H.P.-incubated conditions. After that, a luciferase assay was performed with the cells to get RLU reads using a luminometer as VSV-ΔG is tagged with both GFP and firefly luciferase).

### 3. Pseudovirus testing (VSV-ΔG - SARS CoV2 S spike d21) against Hypericum perforatum (H.P.) or its ingredients (description is exemplary for Hypericum perforatum (H.P.)):

- On day 1, vero cells were seeded overnight (5×10⁴) in DMEM 10% FCS (100µl total volume).
- On day 2, media was replaced by fresh media (100µl DMEM 10% FCS) containing either DMSO (as solvent control) or H.P. (25µg/ml) and incubated at 37°c/ 1h. Meanwhile, the VSV pseudovirus carrying SARS CoV-2 d21 spike protein was incubated with either DMSO (as solvent control) or H.P. (25µg/ml) for 1h at room temperature in DMEM 10% FCS infection mix. After pretreatment of cells and virus, virus infection was done with an MOI of 0.01 at 37°c/ 1h using 40µl infection solution/well. After infection, cells were finally washed and further incubated with regular DMEM 10% FCS without treatment substances for 18-22 hours.
- On day 3, cells were visualized under fluorescent microscope (GFP positive cells are green in color) to get a count of GFP positive cells in both control and H.P.-incubated conditions. After that, a luciferase assay was performed with the cells to get RLU reads using a luminometer (VSV-ΔG is tagged with both GFP and firefly luciferase).

### 4. SARS CoV2 testing against Hypericum perforatum (H.P.):

- On day 1, Vero cells were seeded overnight (5×10⁵) in DMEM 10% FCS (1ml total volume).
- On day 2, media was replaced by a fresh 500µl SARS CoV2-infection DMEM (2% FCS), which contains either DMSO (as solvent control) or H.P. (different concentrations tested) and incubated at 37°c/ 1h. Meanwhile, the SARS CoV-2 virus was incubated with either DMSO (as solvent control) or H.P. (different concentrations tested) for 1h at room temperature in an infection PBS mix. After preincubation of cells and virus, infection was done with 350µl volume (MOI of 0.05) at 37°c/ 1h. After infection, cells were finally washed with PBS and incubated with new infection DMEM (2% FCS) containing DMSO (as solvent control) or H.P. (different concentrations tested) for 24h or 48h. Virus supernatants were collected, frozen at -80°c and standard plaque assay was performed to obtain PFU/ml.

### 5. SARS-CoV2 VOC testing against Hypericum perforatum, and its ingredient Hypericin:

To investigate the impact of Hypericum perforatum and its ingredient Hypericin on SARS-CoV2 Variants of Concern (VOC), three candidate SARS-COV-2 virus variants (B.1.1.7, B.1.351, and B.1.617.2) were selected for full treatment conditions (pre-treatment of cells, pre-treatment of virus, and post-treatment of cells) for either of the two substances:
   - On day 1: Vero cells were seeded overnight in DMEM-10%FCS.
   - On day 2: Old media was replaced by a fresh 500µl SARS-CoV2-infection DMEM (I-DMEM-2%FCS), which contains either DMSO (as solvent control) or Hypericum (15µg/ml) or Hypericin (10ng/ml) and cells were incubated at 37°C/1h. Meanwhile, the SARS-CoV2 VOC virus was incubated with either DMSO (as solvent control) or to be tested substances (indicated concentrations tested) for 1h at room temperature in an infection I-PBS mix.
After pre-incubation of cells and virus, infection was done with 350µl volume (MOI of 0.05) at 37°C /1h. Following infection, cells were finally washed with normal PBS and incubated with new I-DMEM-2%FCS containing either DMSO (as solvent control) or Hypericum perforatum (15µg/ml) or Hypericin (10ng/ml). After 24h infection, virus supernatants were collected, frozen at -80°C and standard plaque assay was performed to determine PFU/ml.
Plaques were counted 3-4 days later.

### 6. Light independent experiments using the composition according to the invention:

- The experiments mentioned herein were also carried out in the dark. Hereto, the cells were left in the dark or in the light after treatment with the compositions according to the invention.

### 7. Virus reduction by hypericin when applied to infected cells:

- In this experiment the cells were treated with only hypericin (250ng/ml) after infection (i.e. without pre-treatment of the virus).

### Results

- MTT assay showed that Hypericum perforatum (H.P.) is not toxic up to at least 25µg/ml (Fig. 1-2). Also, its ingredients Hypericin (Fig.4), Pseudohypericin (Fig. 5), and Hyperforin (Fig. 6) were not toxic up to 1µg/ml, 1µg/ml, and 2µg/ml, respectively. In addition, Procyanidin C1 (Fig. 7) and quercetin-3-O-glucuronid (Fig. 8) and were also not toxic up to 50µM concentration.
- Preliminary results indicated the potential impact of Hypericum perforatum against the VSV pseudovirus (25µg/ml) (Fig. 1-2) or the real SARS CoV-2 virus with low as 25µg/ml showed three log steps reduction and meanwhile higher concentrations resulted in complete inhibition of the virus (Fig.3) Studying the ingredients of Hypericum perforatum, Hypericin and pseudohypericin (1µg/ml) clearly inhibited the VSV pseudovirus to various levels (Fig. 4.5). However, Hyperforin did not have comparable impact (Fig. 6), indicating that it is potentially not involved in the inhibition capability of H.P. on SARS CoV-2. On the other hand, procyanidin C1 and quercetin-3-O-glucuronid did not have any obvious effect on the VSV pseudovirus (no significant inhibition) (Fig. 7-8).

Taken together, the here-depicted results clarify a potential antiviral effect of a plant extract from Hypericum perforatum against SARS CoV-2. A clear and reproducible block of SARS CoV-2 S protein mediated virus entry was shown using the VSV-ΔG SARS CoV-2 S pseudovirus system. In SARS CoV-2 virus infections, extracts of Hypericum perforatum do show strong antiviral capacities in first pilot experiments, which has to be investigated in more depth. Regarding potentially active ingredients within the Hypericum perforatum extract, Hypericin and Pseudohypericin seem to carry such activities against SARS CoV-2 S protein mediated virus binding
- The results shown in Figure 9 confirm that Hypericum perforatum and its ingredient Hypericin are active against the SARS-CoV-2 VOC (Alpha, Beta, and Delta) in the same concentrations as for parental SARS-CoV-2, since virus titers dropped nearly 3-5 log steps in either Hypericum- or Hypericin-treated samples in comparison to DMSO-treated controls.
- The results shown in Figures 10 and 11 (A and B) confirm that the experiments described herein also work in the dark (which corresponds to the clinical situation in the body).
- The results shown in Figure 12 confirm that hypericum, especially hypericin, dramatically lowers the viral load in diseased cells (which corresponds to the therapeutic situation).

## Claims

1. Composition comprising hypericin and/or pseudohypericin for use in a method for treating or preventing an infection with SARS-CoV2 in a human subject in need thereof, wherein said composition does not comprise non-extracted Hypericum perforatum (H.P.) solid plant material.

2. Composition for the use according to claim 1, wherein said composition is enterally or parenterally administered to said human subject.

3. Composition for the use according to claim 1, wherein said composition is administered to mucosa tissue of said human subject.

4. Composition for the use according to claim 3, wherein said mucosa tissue is selected from mucosa of the upper respiratory tract and mucosa from the lower respiratory tract.

5. Composition for the use according to claim 4, wherein said mucosa of the upper respiratory tract is selected from nasal mucosa, oral mucosa and pharyngeal mucosa.

6. Composition for the use according to claim 4, wherein said mucosa of the lower respiratory tract is selected from tracheal mucosa, bronchial mucosa and mucosa of the pulmonary alveoli.

7. Composition for the use according to any of the preceding claims, wherein said hypericin and/or pseudohypericin is comprised by or is an extract of Hypericum perforatum, wherein said composition or extract does not comprise non-extracted Hypericum perforatum (H.P.) solid plant material.

8. Composition for the use according to claim 7, wherein said H.P. extract comprises hypericin at a concentration between about 0.1 w/w% to about 0.3 w/w%.

9. Composition for the use according to any of the preceding claims, which further comprises a solvent for hypericin and/or pseudohypericin, and/or a solvent for an extract of Hypericum perforatum.

10. Composition for the use according to any of the preceding claims, wherein said composition is in the form of a mouth wash, a gargle, a nose spray or nose drops, a mouth or pharyngeal spray, an nasal ointment, e.g. a nasal ointment, a gel, e.g. a nasal gel, an inhalation spray or solution, a nebulizer solution, a lozenge, or a tablet, a capsule, a pill, or an injectable solution.

11. Composition for the use according to any of claims 2 to 10, which comprises an H.P. extract at a concentration of at least about 0.25 µg per ml.

12. Composition for the use according to any of the preceding claims, which comprises hyperforin at a concentration of not more than 5 µg/ml.

13. Composition for the use according to any of the preceding claims, which is a pharmaceutical composition.

14. Composition for the use according to any of the preceding claims, wherein when treating or preventing an infection with SARS-CoV2 in a human subject in need thereof, said SARS-CoV2 comprises, in addition to wild-type SARS-CoV-2, any one of the Alpha, Beta and/or Delta variant of SARS-CoV2, preferably any one of the variant B.1.1.7, B.1.351 and/or B.1.617.2.

15. Mouth wash, gargle solution, nose spray, inhalant, lozenge, chewing gum, injectable solution comprising hypericin and/or pseudohypericin.
